**Europäisches Patentamt**

**European Patent Office**

**Office européen des brevets**

(19)

(11) Veröffentlichungsnummer: **0 000 468**
**A1**

(12) # EUROPÄISCHE PATENTANMELDUNG

(21) Anmeldenummer: **78100043.5**

(22) Anmeldetag: **01.06.78**

(51) Int. Cl.²: **C 07 C 69/80,C 08 K 5/12**

(30) Priorität: **01.06.77 DE 2724609**

(43) Veröffentlichungstag der Anmeldung:
**07.02.79 Bulletin 79/3**

(84) Benannte Vertragsstaaten:
**BE CH DE FR GB**

(71) Anmelder: **BASF Aktiengesellschaft**
**Carl-Bosch-Strasse 38**
**D-6700 Ludwigshafen. (DE)**

(72) Erfinder: **Gehm, Robert, Dr.**
**Mannheimer Strasse 45**
**D-6703 Limburgerhof. (DE)**

(72) Erfinder: **Jarre, Wolfgang, Dr.**
**Limesstrasse 3**
**D-6700 Ludwigshafen-Rheingönheim. (DE)**

(54) Chlor- und estergruppenhaltige Polyole auf Basis von Tetrachlorphtalsäure, deren Herstellung und Verwendung zur Herstellung von flammbeständigen Polyurethan- oder Polyisocyanuratschaumstoffen.

(57) Chlor- und estergruppenhaltige Polyole durch Alkoxylierung eines Tetrachlorphthalsäurehalbesters mit einer Viskosität von 30 bis 20 000 m.Pa.s, gemessen bei 130°C, mit Alkylenoxiden im Aequivalenzverhältnis von Carboxylgruppe zu Alkylenoxid von 1:1 bis 1,5 hergestellt.

Die Erfindungsgemässen Polyole, die Molekulargewichte von 1 000 bis 2 500, Hydroxylzahlen von 168 bis 70, Viskositäten von 50 bis 500 m.Pa.s und Chlorgehalte von 20 bis 35 Gew.%, bezogen auf das Gesamtgewicht, besitzen, eignen sich insbesondere zur Herstellung von flammbeständigen Polyurethan- und Polyisocyanatschaumstoffen, die im Brandfalle eine geringe Rauchgasentwicklung zeigen.

EP 0 000 468 A1

0000468

BASF Aktiengesellschaft                              O. Z. 0050/032522

## Chlor- und estergruppenhaltige Polyole

Die Erfindung betrifft chlor- und estergruppenhaltige Polyole auf Basis von Tetrachlorphthalsäure, deren Herstellung und Verwendung zur Herstellung von flammbeständigen Polyurethan- oder Polyisocyanuratschaumstoffen.

Die Herstellung von flammbeständigen Kunststoffen für den industriellen und privaten Verbrauch gewinnt immer mehr an Bedeutung. Dies gilt besonders auch für geschäumte Kunststoffe auf Polyurethan- und Polyisocyanuratbasis, die als Polyester für Möbel und Autositze oder auch als Isoliermaterialien verwendet werden.

Es sind zahlreiche Verfahren bekannte, Polyurethan- und Polyisocyanuratschaumstoffen eine gewisse Flammwidrigkeit zu verleihen. So wurde vorgeschlagen, den schaumfähigen Mischungen beispielsweise antimon-, bor-, phosphor- und/oder halogenhaltige Verbindungen einzuverleiben. Alle die genannten Verbindungen vermögen den Schaumstoffen eine gewisse Flammresistenz zu verleihen. Nachteilig hierbei ist jedoch, daß die Verwendung großer Mengen dieser Flammschutzmittel zu einer ernsthaften Beeinträchtigung der mechanischen Eigenschaften der Schaumstoffe führt.

M/G1

Es wurde ferner vorgeschlagen, halogenhaltige Polyesterole auf Basis von Tetrabrom- und Tetrachlorphthalsäure zur Herstellung von selbstverlöschenden Polyurethanschaumstoffen zu verwenden. Die halogenhaltigen Polyesterole werden hierbei durch Umsetzung von Polyalkylenoxiden mit Molekulargewichten von 100 bis 10 000 mit Tetrahalogenphthalsäureanhydriden und anschließender Alkoxylierung der erhaltenen Halbester mit Alkylenoxiden erhalten. Entsprechende Produkte werden beispielsweise in den US-Patentschriften 3,455,886 und 3,585,185 sowie in der Publikation "Tetrabromphthalsäureanhydrid in feuerbeständigen Polyurethanschäumen" von Pape et al beschrieben (SPE 26. An. Techn. Conf. Papers, Band 14, Seite 695, 1968).

Nachteilig an den beschriebenen bromhaltigen Polyesterolen ist neben dem hohen Preis eine unerwünscht hohe Rauchgasdichte, die sich im Brandfalle aus derartigen Kunststoffen entwickelt. Wird anstelle von Tetrabromphthalsäureanhydrid das entsprechende Chlorderivat verwendet, so wird der Halogengehalt der Polyesterole und dadurch die flammhemmende Wirkung verringert und gleichzeitig steigt die Viskosität der erhaltenen Endprodukte so stark an, daß sie mit den anderen Komponenten der schaumfähigen Polyurethanmischung nicht mehr oder nur mit Hilfe eines großen apparativen Aufwands homogen mischbar sind.

Aufgabe der vorliegenden Erfindung war es Polyole zu entwickeln, die in üblichen Verarbeitungsvorrichtungen mit anderen Ausgangsstoffen zur Herstellung von Polyurethan- und Polyisocyanuratschaumstoffen homogen gemischt werden können und die den Schaumstoffen im Brandfall eine mit dem Stand der Technik zumindest vergleichbare Flammschutzwirkung bei deutlich verringerter Rauchgasdichte verleihen.

0000468

Diese Aufgabe wurde gelöst mit Hilfe von chlor- und estergruppenhaltigen Polyolen, die durch Alkoxylierung eines Tetrachlorphthalsäurehalbesters mit einer Viskosisät von 30 bis 20 000 m.Pa.s, gemessen bei 130°C, mit Alkylenoxiden im Äquivalenzverhältnis von Carboxylgruppe zu Alkylenoxid von 1:1 bis 1,5 hergestellt werden, wobei der Tetrachlorphthalsäurehalbester seinerseits erhalten wird durch Umsetzung von Tetrachlorphthalsäureanhydrid mit

a) einer Mischung aus 2- bis 6-wertigen Alkoholen mit Molekulargewichten von 60 bis 300 oder

b) einer Mischung aus 2- bis 6-funktionellen hydroxylgruppenhaltigen Polyäthern mit einem Molekulargewicht von 100 bis 800 oder

c) einer Mischung aus einem 2- bis 6-wertigen Alkohol mit einem Molekulargewicht von 60 bis 300 und einem 2- bis 6-funktionellen hydroxylgruppenhaltigen Polyäther mit einem Molekulargewicht von 100 bis 800

mit der Maßgabe, daß die Mischung eine Funktionalität von 2,8 bis 4,5 besitzt und das Äquivalenzgewichtsverhältnis von Hydroxyl- zu Anhydridgruppe 1,1 bis 1:1 ist.

Überraschenderweise weisen die aus den erfindungsgemäßen chlor- und estergruppenhaltigen Polyolen hergestellte Schaumstoffe neben der bereits genannten guten Flammresistenz und stark verminderter Rauchentwicklung im Brandfalle außerdem eine sehr gute Hydrolysebeständigkeit und somit erhöhte Stabilität auf.

Zur Herstellung der erfindungsgemäßen chlor- und estergruppenhaltigen Polyole werden Tetrachlorphthalsäurehalbester mit einer Viskosität von 30 bis 20 000 m.Pa.s, vorzugsweise

0000468

von 400 bis 5 000 m.Pa.s, gemessen bei 130°C mit Alkylenoxiden alkoxiliert.

Als Alkylenoxide kommen Äthylenoxid und vorzugsweise Propylenoxid in Betracht. In Abhängigkeit von der gewünschten Reaktivität der chlor- und estergruppenhaltigen Polyole kan es vorteilhaft sein Mischungen aus Äthylen- und Propylenoxid zu verwenden, wobei die Alkylenoxide in breiten Grenzen, beispielsweise in den Molverhältnissen Äthylenoxid zu Propylenoxid von 95:5 bis 5:95, vorzugsweise von 50:50 bis 20:80 variiert werden können.

Die Alkoxilierungsreaktion wird bei Temperaturen von 80 bis 160°C, vorzugsweise von 100 bis 135°C, bei Normaldruck oder vorzugsweise unter erhöhtem Druck von 1,5 bis 10 bar vortei: hafterweise in Gegenwart von unter den Reaktionsbedingungen inerten Gasen, wie Stickstoff, Helium u.a. oder deren Gemischen durchgeführt. Erfindungsgemäß wird der Tetrachlorphthalsäurehalbester und das Alkylenoxid in solchen Mengen zur Reaktion gebracht, daß das Äquivalenzverhältnis von Carboxylgruppe zu Alkylenoxid 1:1 bis 1,5, vorzugsweise 1:1 bis 1,2 beträgt. Um eine nachträgliche Reinigung der Endprodukte zu vermeiden, wird die Alkoxilierung vorzugsweise in Abwesenheit von Katalysatoren durchgeführt.

Wie bereits dargelegt wurde, sind zur Herstellung der chlor- und estergruppenhaltigen Polyole nur Tetrachlorphthalsäurehalbester mit Viskositäten von 30 bis 20 000 m.Pa.s, gemessen bei 130°C, verwendbar. Derartige Tetrachlorphthalsäurehalbester werden hergestellt durch Umsetzung von Tetrachlorphthalsäureanhydrid mit a) einer Mischung aus zwei 2- bis 6-wertigen, vorzugsweise 2- bis 4-wertigen Alkoholen mit Molekulargewichten von 60 bis 300, vorzugsweise von 60 bis 183, oder b) einer Mischung aus zwei 2- bis 6-, vorzugsweise 2- bis 4,5-funktionellen hydroxylgruppenhaltigen Poly-

0000468

äther mit einem Molekulargewicht von 100 bis 800, vorzugsweise 250 bis 650 oder c) vorzugsweise mit einer Mischung aus einem 2- bis 6-wertigen Alkohol mit einem Molekulargewicht von 60 bis ⪰300, vorzugsweise 60 bis 183 und einem 2- bis 6-, vorzugsweise 2 bis 4,5-funktionellen hydroxylgruppenhaltigen Polyäther mit einem Molekulargewicht von 100 bis 800, vorzugsweise von 250 bis 650. Erfindungswesentlich ist, daß die Mischungen aus Alkoholen, hydroxylgruppenhaltigen Polyäthern oder aus einem Alkohol und einem hydroxylgruppenhaltigen Polyäther eine Funktionalität (f) von 2,8 bis 4,5, vorzugsweise 2,8 bis 3 besitzen, wobei die Funktionalität berechnet wird mit Hilfe der Formel

$$f = \frac{n_1 \cdot f_1 + n_2 \cdot f_2}{n_1 + n_2}$$

in der bedeuten $f_1$ und $f_2$ die Funktionalität und $n_1$ und $n_2$ die Anzahl der Mole der Mischungskomponenten.

Zur Herstellung der Tetrachlorphthalsäurehalbester werden die Ausgangskomponenten bei Temperaturen von 100 bis 180°C, vorzugsweise von 110°C bis 140°C bei Normaldruck oder gegebenenfalls bei Drucken von 1,5 bis 7 bar in Gegenwart von üblichen Inertgasen in solchen Mengen verestert, daß das Äquivalenzverhältnis von Hydroxylgruppen der Mischung zu Anhydridgruppe 1,1 bis 1:1, vorzugsweise ungefähr 1:1 beträgt. Die Veresterungsreaktion zum Tetrachlorphthalsäurehalbester kann ebenso wie die anschließende Alkoxilierungsreaktion zum -Diester gegebenenfalls in Gegenwart von inerten Lösungsmitteln, wie Toluol, Dioxan u.a. durchgeführt werden.

Zur Herstellung der Alkoholmischung, der hydroxylgruppenhaltigen Polyäthermischung bzw. der Mischung aus Alkohol und hydroxylgruppenhaltigen Polyäthern kommen 2- bis 6-wer-

Eige Alkohole in Betracht. Im einzelnen seien beispielhaft genannt: 2-wertige Alkohole, wie Äthylenglykol, Propylenglykol-1,2, Propylenglykol-1,3, Butandiol-1,4 und Hexandiol-1,6; 3-wertige Alkohole, wie Glycerin, Trimethylolpropan Butantriol und Hexantriol und 4- bis 6-wertige Alkohole, wie Pentaerythrit und Sorbit. Vorzugsweise verwendet werden Glycerin und niedermolekulare Diole, wie Äthylen- oder Propylenglykol-1,2.

Als hydroxylgruppenhaltige Polyätherole haben sich solche mit Funktionalitäten von 2 bis 6, vorzugsweise von 2 bis 4, und Molekulargewichten von 100 bis 800, vorzugsweise 250 bis 650 besonders bewährt. Geeignete hydroxylgruppenhaltige Poly äther können dadurch hergestellt werden, daß man ein oder mehrere gegebenenfalls substituierte Alkylenoxide mit 2 bis 4 Kohlenstoffatomen im Alkylenrest mit einem Startermolekül das mehrere aktive Wasserstoffatome gebunden enthält, umsetzt. Als Alkylenoxide seien beispielhaft genannt: 1,2-Butylenoxid, Styroloxid, Epichlorhydrin und vorzugsweise Äthylenoxid und 1,2-Propylenoxid. Die Alkylenoxide können einzeln, alternierend nacheinander oder als Mischungen verwendet werden. Als Startermoleküle kommen beispielsweise in Betracht: Wasser; Phosphorsäure; Ammoniak; Hydrazine; aliphatische, cycloaliphatische und aromatische Amine, wie Äthylendiamin, N,N-Dimethyläthylendiamin, Tetramethylendiamin, Hexamethylendiamin, Diäthylentriamin, o-, m-, p-Phenylendiamin, 2,4- und 2,6-Diaminotoluol, 2,4'-, 2,2'-, 4,4'-Diamino-diphenylmethan und Melamin; Aminoalkohole, wie Äthanolamin, Di- und Triäthanolamin; Polycarbonsäuren, wie Adipinsäure und Terephthalsäure und vorzugsweise Polyhydroxylverbindungen, wie Äthylenglykol, Diäthylenglykol, Propandiol-1,2, Propandiol-1,3, Butandiol-1,4, Butandiol-1,3, Hexandiol-1,6, Butin-2-diol-1,4, Glycerin, Trimethylolpropan, Hexantriol-2,4,6, Pentaerythrit, Sorbit und Saccharose.

Besonders bewährt haben sich und daher vorzugsweise verwendet werden beispielsweise folgende hydroxylgruppenhaltige Polyätherole: ein trifunktionelles Addukt auf Basis Glycerin-Propylenoxid mit einem mittleren Molekulargewicht von 420, ein tetrafunktionelles Addukt auf Basis Äthylendiamin-Propylenoxid mit einem mittleren Molekulargewicht von 292, ein Polypropylenglykol mit einem mittleren Molekulargewicht von 450 und ein 4,3-funktionelles Addukt auf Basis Saccharose-Wasser-Propylenoxid mit einem mittleren Molekulargewicht von 600.

Geeignete Mischungen aus 2- bis 6-wertigen Alkoholen, hydroxylgruppenhaltigen Polyäthern bzw. einem 2- bis 6-wertigen Alkohol und einem hydroxylgruppenhaltigen Polyäther sind beispielsweise Mischungen mit einer mittleren Funktionalität von 3 aus Trimethylolpropan und Glycerin; Glycerin und einem Glycerin-Propylenoxid-Addukt; Trimethylolpropan und einem Glycerin-Propylenoxid-Addukt; Sorbit und einem Polypropylenglykol; Pentaerythrit und einem Polypropylenglykol, Äthylenglykol und einem Saccharose-Wasser-Propylenoxid-Addukt; Hexandiol-1,6 und einem Saccharose-Wasser-Propylenoxid-Addukt und einem Addukt auf Basis Äthylendiamin-Propylenoxid mit einem Polypropylenglykol.

Die erfindungsgemäßen chlor- und estergruppenhaltigen Polyole, die Molekulargewichte von 1000 bis 2500, vorzugsweise von 1200 bis 2000, Hydroxylzahlen von 168 bis 70, vorzugsweise von 140 bis 84, Viskositäten von 50 bis 500 m.Pa.s, vorzugsweise von 75 bis 350 m.Pa.s, gemessen bei 130°C und Chlorgehalte von 20 bis 35 Gew.%, vorzugsweise von 25 bis 30 Gew.%, bezogen auf das Gesamtgewicht besitzen, eignen sich insbesonders zur Herstellung von flammbeständigen Polyurethan- und Polyisocyanuratschaumstoffen, die im Brandfalle eine geringe Rauchgasentwicklung zeigen.

Die Herstellung von Polyurethan- und Polyisocyanuratschaumstoffen ist aus der umfangreichen Fachliteratur bekannt. Zur Herstellung der Schaumstoffe können, neben anderen Ausgangskomponenten, wie Polyisocyanaten, Kettenverlängerungsmitteln, Katalysatoren, Hilfs- und Zusatzstoffen, die erfindungsgemäßen chlor- und estergruppenhaltigen Polyole allein oder in Form von Mischungen mit üblichen bekannten Polyhydroxylverbindungen, beispielsweise niedermolekularen mehrwertigen Alkoholen, Polyäther- und/oder Polyesterolen verwendet werden.

Als Mischungskomponente geeignet sind beispielsweise niedermolekulare mehrwertige Alkohole, wie Äthylenglykol, Diäthylenglykol, Propylenglykole, Butandiol-1,4, Glycerin und Zuckeralkohole.

Als Kettenverlängerungsmittel kommen vorzugsweise Diol, wie Äthylenglykol, Diäthylenglykol, Butandiol-1,4 und Hexandiol in Betracht.

Geeignete Polyätherole können - wie bereits dargelegt wurde - dadurch hergestellt werden, daß man ein oder mehrere Alkylenoxide mit 2 bis 4 Kohlenstoffatomen im Alkylenrest mit einem Startermolekül, das mehrere aktive Wasserstoffatome gebunden enthält, umsetzt. Geeignete Alkylenoxide sind z.B. Äthylenoxid, 1,2-Propylenoxid, Epichlorhydrin und 1,2-Butylenoxid. Die Alkylenoxide können einzeln, alternierend nacheinander oder als Mischungen verwendet werden. Als Startermoleküle kommen beispielsweise in Betracht: Wasser; Phosphorsäure; Amine wie Ammoniak, Hydrazin, Äthylendiamin, Hexamethylen-diamin, Toluylendiamin, Diamino-diphenylmethan und Melamin; Aminoalkohole, wie Mono- und Diäthanolamin, Polycarbonsäuren, wie Adipinsäure und Terephthalsäure und Polyhydroxylverbindungen, wie Äthylenglykol, Propylenglykol, Diäthylenglykol, Glyzerin, Trimethylolpropan, Pentaerythrit, Sorbit und Saccharose. Die Polyalkylenäther, die geradkettig, teilverzweigt oder verzweigt sein können, besitzen Molekulargewichte von 300 bis 10 000, vorzugsweise von 400 bis 5 000, und Hydroxylzahlen von 30 bis 800, vorzugsweise von 35 bis 400.

Geeignete Polyesterpolyole können beispielsweise aus Dicarbonsäuren und mehrwertigen Alkoholen hergestellt werden. Als Dicarbonsäuren kommen beispielsweise in Betracht: aliphatische Dicarbonsäuren, wie Bernsteinsäure, Glutarsäure, Adipinsäure, Korksäure, Azelainsäure und Sebacinsäure und aromatische Dicarbonsäure, wie Phthalsäure, Isophthalsäure und Terephthalsäure. Die Säuren können einzeln oder als Gemisch verwendet werden. Zur Herstellung der Polyesterole kann es gegebenenfalls vorteilhaft sein, anstelle der Carbonsäuren die entsprechenden Carbonsäurederivate, wie Carbonsäureester mit 1 bis 4 Kohlenstoffatomen im Alkoholrest, Carbonsäureanhydride oder Carbonsäurechloride zu verwenden. Beispiele für mehrwertige Alkohole sind Glykole, wie Äthylenglykol, Diäthylenglykol, Butandiol-1,4, Pentandiol-1,5, Hexandiol-1,6, Decandiol-1,10, 2,2-Dimethylpropandiol-1,3 und 2,2,4-Trimethylpentandiol-1,3, Triole, wie Glycerin und Trimethylolpropan und Polyole, wie Pentaerythrit, Sorbit und Saccharose. Je nach den gewünschten Eigenschaften können die Polyole allein oder als Mischungen in verschiedenen Mengen verwendet werden. Die Polyesterpolyole, die gerad- oder verzweigtkettig, di- oder polyfunktionell aufgebaut sein können, besitzen Molekulargewichte von 500 bis 5 000, vorzugsweise von 800 bis 3 000 und Hydroxylzahlen von 30 bis 500, vorzugsweise von 40 bis 250.

Als Polyisocyanate kommen vorzugsweise aromatische Di- und Polyisocyanate in Betracht. Im einzelnen seien beispielhaft genannt: aromatische Diisocyanate, wie 1,3- bzw. 1,4-Pheny-

Iendiisocyanat, 1,5- bzw. 1,8-Naphthylen-diisocyanat, 3,3'- bzw. 4,4'-Diphenyldiisocyanat und vorzugsweise 2,4- und/oder 2,6-Toluylendiisocyanat und 2,2'-, 2,4'- und/oder 4,4'-Diphenylmethan-diisocyanat sowie Polyisocyanate, wie 2,4,6-Toluylentriisocyanat und Polyphenyl-polymethylenpolyisocyanat, die durch Kondensation von Anilin mit Formaldehyd in Gegenwart von Säuren als Katalysatoren und anschliessende Phosgenierung der erhaltenen Basen hergestellt werden. Verwendet werden vorzugsweise die käuflich erwerbbaren aromatischen 2,4- und/oder 2,6-Toluylendiisocyanate sowie Mischungen aus Di- und Polyphenyl-polymethylen-polyisocyanaten (Roh-MDI). Die Produkte können einzeln oder als Mischungen eingesetzt werden.

Zur Herstellung der Polyurethanschaumstoffe werden die Hydroxylgruppen aufweisenden Verbindungen und Polyisocyanate in solchen Mengenverhältnissen verwendet, daß pro Äquivalent Hydroxylgruppe 0,9 bis 1,1, vorzugsweise 1,01 bis 1,05 Äquivalente Isocyanatgruppen vorliegen, während zur Herstellung der Polyisocyanuratschaumstoffe pro Hydroxylgruppe 3 bis 60, vorzugsweise 5 bis 30 NCO-Gruppen zur Anwendung gelangen.

Zur Beschleunigung der Cyclisierung und Polymerisation der Di- und/oder Polyisocyanate sowie die Umsetzung zwischen den hydroxylgruppenhaltigen Verbindungen und Di- und/oder Polyisocyanaten werden den schaumfähigen Mischungen zweckmäßig übliche Tri- bzw. Polymerisationskatalystoren und/oder Polyurethankatalysatoren einverleibt.

Als Tri- bzw. Polymerisationskatalysatoren seien beispielhaft genannt: starke Basen, wie quaternäre Ammoniumhydroxide, beispielsweise Benzyltrimethylammoniumhydroxid, Alkalimetallhydroxide, beispielsweise Natrium- oder Kaliumhydroxid, Alkalimetallalkoxide, beispielsweise Natrium-

methylat und Kaliumisopropylat, Trialkylphosphine, beispielsweise Triäthylphosphin, Alkylaminoalkylphenole, beispielsweise 2,4,6-Tris(dimethylaminomethyl)-phenol, 3- und/
oder 4-substituierte Pyridine, beispielsweise 3- oder
4-Methylpyridin, metallorganische Salze, beispielsweise
Tetrakis(hydroxyäthyl)natriumborat, Friedel-Crafts-Katalysatoren, beispielsweise Aluminiumchlorid, Eisen(III)chlorid, Borfluorid und Zinkchlorid und Alkalimetallsalze von
schwachen organischen Säuren und Nitrophenolaten, beispielsweise Kaliumformiat, Kaliumoctoat, Kalium-2-äthyl-hexoat,
Kaliumbenzoat, Natriumpikrat und Phthalimidkalium. Geeignete Trimerisierungskatalysatoren sins ferner die stark
basischen N,N',N"-Tris-(dialkylaminoalkyl)-s-hexahydro-
triazine, beispielsweise das N,N',N"-Tris-(dimethyl-3-ami-
nopropyl)-s-triazin und 2,4,6-Tris-(diäthanolamino)-s-triazin sowie Mischungen aus den genannten Triazinderivaten
und deren Carbonsäureaddukten.

Zur Beschleunigung der Polyurethanbildung kommen beispielsweise in Betracht: tertiäre Amine, wie 1,4-Diazabicyclo-
(2,2,2)-octan, N,N-Dimethylbenzylamin, Triäthylamin,
Pyridin, N-Methylmorpholin, N-Dimethylaminoäthylmorpholin
und N-Dimethylaminopropyl-morpholin, Metallsalze wie
Eisen-II-chlorid, Zinkchlorid, Zinn-II-salze und Dibutylzinndilaurat und Mischungen aus tertiären Aminen und Metallsalzen.

Zur Herstellung der Schaumstoffe wird als Treibmittel üblicherweise Kohlendioxid verwendet, das durch die Umsetzung
von Isocyanaten mit Wasser entsteht. Die Wassermengen, die
zweckmäßigerweise verwendet werden können, betragen 0,1
bis 2 %, bezogen auf das Gewicht an Polyisocyanat.

Andere verwendbare Treibmittel sind niedrigsiedende Flüssigkeiten, die unter dem Einfluß der exothermen Polymeri- sationsreaktion verdampfen. Geeignet sind Flüssigkeiten, welche gegenüber dem organischen Polyisocyanat inert sind und Siedepunkte von nicht über 100°C bei Atmosphärendruck, vorzugsweise zwischen -40 und +50°C aufweisen. Beispiele derartiger, vorzugsweise verwendeter Flüssigkeiten sind halogenierte Kohlenwasserstoffe, wie Methylenchlorid, Trichlorfluormethan, Dichlordifluormethan, Dichlormonofluormethan, Dichlortetrafluoräthan und 1,1,2-Trichlor-1,2,2-trifluoräthan. Auch Gemische dieser niedrig siedenden Flüssigkeiten untereinander und/oder mit anderen substituierten oder unsubstituierten Kohlenwasserstoffen können verwendet werden.

Die zweckmäßigste Menge an niedrig siedender Flüssigkeit zur Herstellung von Halbhart- und Hartschaumstoffen hängt ab von der Schaumdichte, die man erreichen will, sowie gegebenenfalls von der Mitverwendung von Wasser. Im allgemeinen liefern Mengen von 5 bis 40 Gew.%, bezogen auf 100 Gewichtsteile organisches Polyisocyanat, zufriedenstellende Ergebnisse.

Der Reaktionsmischung können auch noch Hilfsmittel und Zusatzstoffe einverleibt werden. Genannt seien beispielsweise Stabilisatoren, Hydrolysenschutzmittel, Porenregler, fungistatisch und bakteriostatisch wirkende Substanzen, Farbstoffe, Pigmente, Füllstoffe, oberflächenaktive Stoffe, Weichmacher und andere Flammschutzmittel.

In Betracht kommen beispielsweise oberflächenaktive Substanzen, welche zur Unterstützung der Homogenisierung der Ausgangsstoffe dienen und gegebenenfalls auch geeignet sind, die Zellstruktur der Schaumstoffe zu regulieren. Genannt seien beispielhaft Siloxan-Oxalkylen-Mischpolymerisate und andere Organopolysiloxane, oxäthylierte Alkylphenole, oxäthylierte Fettalkohole, Paraffinöle, Rizinusöl- bzw. Rizinolsäure-Ester und Türkischrotöl, die

0000468

In Mengen von 0,2 bis 6 Gewichtsteilen pro 100 Gewichtsteile Polyisocyanat angewandt werden.

Es kann auch vorteilhaft sein, einen Weichmacher in das Reaktionsgemisch einzubeziehen, so daß die Neigung zur Sprödigkeit in den Produkten verringert wird. Es können übliche Weichmachungsmittel verwendet werden, doch ist es besonders zweckmäßig, solche Mittel zu verwenden, die Phosphor und/oder Halogenatome enthalten und dadurch die Flammfestigkeit der Kunststoffe zusätzlich vergrößern. Zu solchen Mitteln gehören Trikresylphosphat, Tris-2-chloräthylphosphat, Trischlorpropylphosphat und Tris-2,3-dibrompropylphosphat.

Außer den bereits genannten halogensubstituierten Phosphaten können auch anorganische Flammschutzmittel, wie Antimontrioxid, Arsenoxid, Ammoniumphosphat und Calciumsulfat zum Flammfestmachen der Polyurethan- oder Polyisocyanuratschaumstoffe verwendet werden. Im allgemeinen hat es sich als vorteilhaft erwiesen 5 bis 50 Gewichtsteile, vorzugsweise 5 bis 25 Gewichtsteile der genannten Flammschutzmittel für jeweils 100 Gewichtsteile an organischem Polyisocyanat zu verwenden.

Die Polyurethan- und Polyisocyanuratschaumstoffe können nach dem Präpolymer- und vorzugsweise nach dem one-shot-Verfahren hergestellt werden. Hierzu werden die Polyisocyanate mit den hydroxylgruppenhaltigen Verbindungen, Katalysatoren und gegebenenfalls Kettenverlängerungsmitteln, Hilfs- und Zusatzstoffen bei Temperaturen von 10 bis 80, vorzugsweise 15 bis 40°C in üblichen Vorrichtungen intensiv gemischt und danach läßt man die schaumfähige Mischung aufschäumen.

Die unter Mitverwendung der erfindungsgemäßen chlor- und estergruppenhaltigen Polyole hergestellten Polyurethan- und Polyisocyanuratschaumstoffe besitzen Dichten von 15

0000468

bis 400 kg/m$^3$, vorzugsweise 30 bis 80 kg/m$^3$ und zeichnen sich durch eine gleichmäßige feinzellige Porenstruktur, geringe Sprödigkeit, ausgezeichnete mechanische Eigenschaften und eine sehr gute Thermostabilität und Flammbeständigkeit bei geringer Rauchgasdichte aus. Die Produkte können als Isoliermaterial verwendet werden.

Beispiel 1

Herstellung eines chlor- und estergruppenhaltigen Polyols

In einem für Oxalkylierungen geeigneten Reaktor werden 69,6 kg eines Polyätherpolyols (OH-Zahl 400), hergestellt aus Glycerin und Propylenoxid, mit 15,43 kg Glycerin sorgfältig unter Stickstoffatmosphäre vermischt. Das Polyätherpolyol/Glycerin-Gemisch wird mit 265,28 kg Tetrachlorphthalsäureanhydrid versetzt und unter Rühren 3 Stunden bei 130$^o$C umgesetzt. Der erhaltene Tetrachlorphthalsäurehalbester besitzt eine Viskosität von 2000 m.Pa.s bei 130$^o$C.

Anschließend wird der Stickstoffdruck auf 0,5 bar eingestellt und bei 130$^o$C kontinuierlich 97,04 kg Propylenoxid hinzugefügt. Man läßt das Propylenoxid abreagieren bis der Druck einen konstanten Wert erreicht hat, entspannt und gewinnt ein Reaktionsgut mit den folgenden Kennzahlen:

| | |
|---|---|
| OH-Zahl | 143 |
| Säurezahl | 0,1 |
| H$_2$O % | 0,03 |
| Chlor % gef. | 30,1 |
| th. | 29,46 |
| Viskosität | |
| 75$^o$C | 5 860 m.Pa.s |

Der Kesselinhalt kann anschließend bei $130^{\circ}$C mit 210,5 kg Tris-ß-chloräthylphosphat verdünnt werden. Die Kennzahlen der 650 kg Lösung sind:

| | |
|---|---|
| OH-Zahl | 86 |
| Säurezahl | 0,2 |
| $H_2O$ % | 0,03 |
| Chlor % gef. | 32,8 |
| th. | 31,8 |
| Phosphor % gef. | 3,5 |
| th. | 3,46 |
| Viskosität | |
| $25^{\circ}$C | 13 900 m.Pa.s |

### Beispiele 2 bis 9 und Vergleichsbeispiele A bis F

Zur Herstellung von Tetrachlorphthalsäurehalbester werden die in Tabelle 1 und 2 zusammengefaßten Mischungen aus zwei 2- bis 6-wertigen Alkoholen, zwei hydroxylgruppenhaltigen Polyäthern und einem 2- bis 6-wertigen Alkohol und einem hydroxylgruppenhaltigen Polyäther, die eine mittlere Funktionaltät von 3 besitzen, mit 401,6 Gewichtsteilen Tetrachlorphthalsäureanhydrid unter Rühren bei $130^{\circ}$C in 3 Stunden in die entsprechenden Halbester übergeführt.

Die erhaltenen Tetrachlorphthalsäurehalbester, die Viskositäten von 400 bis 13 500 m.Pa.s. bei $130^{\circ}$C besitzen, können analog den Angaben von Beispiel 1 in die erfindungsgemäßen chlor- und estergruppenhaltigen Polyole übergeführt werden.

Tabelle 1

| Bsp. | Mischungen aus | | Tetrachlorphthalsäure-halbester | | Propylen-oxid | chlor- und estergruppen-haltiges Polyol | | |
|---|---|---|---|---|---|---|---|---|
| | Gew.Tle. | Art | Viskosität m.Pa.s 130°C | Chlorgehalt Gew.% | Gew.Tle. | OH-zahl | Säure-zahl | Viskosität[+] m.Pa.s 125°C |
| 2 | 23 105 | Glycerin und Polyätherol I | 2 000 | 37,5 | 145 | 125 | 0,1 | 180 |
| 3 | 33,6 105 | Trimethylolpropan und Polyätherol I | 2 000 | 36,4 | - | - | - | - |
| 4 | 73,1 112,5 | Polyätherol II und Polyätherol III | 13 500 | 33,5 | 146 | 143 | 0,01 | 480 |
| 5 | 33,6 23,3 | Trimethylolpropan und Glycerin | 3 200 | 42,9 | - | - | - | - |
| 6 | 22,6 168,8 | Sorbit und Polyätherol III | 500 | 33,1 | - | - | - | - |
| 7 | 34 113 | Pentaerythrit und Polyätherol III | 2 000 | 35,9 | 145 | 120 | 0,2 | 300 |
| 8 | 17,7 130,6 | Äthylenglykol und Polyätherol IV | 1 200 | 36,3 | 146 | 141 | 0,2 | 250 |
| 9 | 59,1 130,6 | Hexandiol-1.6 und Polyätherol IV | 400 | 33,3 | - | - | - | - |

Polyätherol    I: Addukt auf Basis Glycerin-Propylenoxid            mittl. Molekulargewicht 420, f = 3
Polyätherol   II: Addukt auf Basis Äthylendiamin-Propylenoxid  mittl. Molekulargewicht 292, f = 4
Polyätherol  III: Polypropylenglykol                                         mittl. Molekulargewicht 450, f = 2
Polyätherol   IV: Addukt auf Basis Saccharose-Wasser-           mittl. Molekulargewicht 600, f = 4,3
                          Propylenoxid
[+] gemessen mit:  ICI Kegel-Platte-Viskometer, hergestellt von Epprecht Instruments und Controls

Tabelle 2

| Vergleichs-beispiel | Mischungen aus | | Tetrachlorphthal-säurehalbester Viskosität m.Pa.s. 130°C |
|---|---|---|---|
| | Gew.Tle. | Art | |
| A | 15,5 | Äthylenglykol und | fest |
| | 73,1 | Polyätherol II | |
| B | 29,5 | Hexandiol-1,6 und | fest |
| | 73,1 | Polyätherol II | |
| C | 34 | Pentaerythrit und | fest |
| | 15,5 | Äthylenglykol | |
| D | 34 | Pentaerythrit und | fest |
| | 29,5 | Hexandiol-1,6 | |
| E | 22,8 | Sorbit und | fest |
| | 20,8 | Äthylenglykol | |
| F | 22,8 | Sorbit und | fest |
| | 44,3 | Hexandiol-1,6 | |

Die Tetrachlorphthalsäurehalbester, hergestellt aus Mischungen gemäß Vergleichsbeispiele A bis F sind bei 130°C fest und daher zur Überführung in die erfindungsgemäßen Polyole unbrauchbar.

Beispiele 10 bis 11

Zur Herstellung von Polyisocyanuratschaumstoffen werden

    102     Gew.Tle. eines erfindungsgemäßen chlor- und ester-
                         gruppenhaltigen Polyols

     48     Gew.Tle. Tris-ß-chloräthylphosphat

1,2 Gew.Tle. eines Schaumstabilisators auf Polysiloxanbasis (DC 193)

3 Gew.Tle. Kaliumformiat 35 gew.%-ig in Äthylenglykol

80 Gew.Tle. Monofluortrichlormethan und

240 Gew.Tle. eines Gemisches aus Diisocyanato-diphenylmethanen und Polyphenyl-polymethylen-
polyisocyanaten (Roh-MDI)

intensiv bei Raumtemperatur gemischt und anschließend läßt
man die schaumfähige Mischung aufschäumen.

Die eingesetzten erfindungsgemäßen Polyole und die erhaltenen mechanischen Eigenschaften der Polyisocyanurat-Schaumstoffe sind in Tabelle 3 zusammengefaßt.

Vergleichsbeispiel G

Verfährt man analog den Angaben der Beispiele 10 und 11, verwendet jedoch anstelle des erfindungsgemäßen Polyols ein
handelsübliches Polyesterol, das Tetrabromphthalsäure einkondensiert enthält, so erhält man, wie Tabelle 2 zeigt,
einen Polyisocyanuratschaumstoff mit höherer Rauchgasdichte
nach der EMPA-Norm.

0000469

Tabelle 3

| Beispiele/Vergleichsbeispiel | 10 | 11 | G |
|---|---|---|---|
| erfindungsgemäßes Polyol | gemäß Beisp. 1 | gemäß Beisp. 4<br>gemessen / ber. [+] | Handelsprodukt auf Basis Tetrabrom-phthalsäure |
| mechanische Eigenschaften: | | | |
| Biegefestigkeit nach DIN 53 422 (kp/cm$^2$) | 2,30 | 1,8/2,4 | 2,39 |
| Durchbiegung nach DIN 53 423 (mm) | 8,8 | 10,5 | 9,5 |
| Druckfestigkeit nach DIN 53 421 (kp/cm$^2$) | 1,4 | 0,85/1,23 | 1,2 |
| Startzeit (sec) | 75 | 34 | 70 |
| Steigezeit (sec) | 240 | 240 | 300 |
| Raumgewicht (g/l) | 30 | 23,4/30,0 | 31 |
| Brandverhalten nach EMPA-Norm | V, 2 | V, 2 | V, 1 |

+ Die gemessenen Meßwerte wurden umgerechnet auf eine Dichte von 30 g/l

0000468

Beispiel 12 bis 20

Zur Herstellung von Polyurethan-Hartschaumstoffen wird eine Mischung aus

einem Polyätherol oder Polyesterol,
einem erfindungsgemäßen chlorhaltigen Polyol,
einem Schaumstabilisator auf Basis eines Siloxan-Oxalkylen-
    Mischpolymerisats (Handelsprodukt DC 190 der Dow
    Dow Corning),
einem Aminkatalysator,
einer oberflächenaktiven Verbindung auf Ricinusölbasis
    (Zusatzmittel SM der Bayer AG) und
einem Treibmittel oder Treibmittelgemisch
mit
einer Mischung aus Diphenylmethan-diisocyanaten und Polyphenyl-polymethylen-polyisocyanaten (Roh-MDJ) bei $25^{\circ}$C
intensiv gemischt und anschließend aufschäumen gelassen.

Die Art und Mengen der verwendeten Ausgangskomponenten, die Start- und Steigzeiten sowie die mechanischen Eigenschaften der erhaltenen Hartschaumstoffe sind in Tabellen 4 und 5 zusammengefaßt.

Tabelle 4

| Beispiele | | 12 | 13 | 14 | 15 | 16 | 17 | 18 | 19 | 20 |
|---|---|---|---|---|---|---|---|---|---|---|
| Polyätherol auf Basis Sucrose. Glycerin, Propylenoxid (OH-Zahl 380) | [Teile] | 40,3 | 40,3 | 40,3 | - | - | - | - | - | - |
| Polyätherol auf Basis Äthylendiamin, Propylenoxid (OH-Zahl 420) | [Teile] | - | - | - | 37 | 37 | 37 | - | - | - |
| Polyesterol auf Basis Bernstein-Glutar-Adipinsäuregemisch und Isopropanolaminen | [Teile] | - | - | - | - | - | - | 37 | 37 | 37 |
| erfindungsgemäße chlorhaltige Polyole gemäß Beispiel 1 | [Teile] | 40,3 | - | - | 37 | - | - | 37 | - | - |
| Beispiel 7 | [Teile] | - | 40,3 | - | - | 37 | - | - | 37 | - |
| Beispiel 8 | [Teile] | - | - | 40,3 | - | - | 37 | - | - | 37 |
| DC 190 | [Teile] | 0,8 | 0,8 | 0,8 | 0,7 | 0,7 | 0,7 | 0,7 | 0,7 | 0,7 |
| Dimethyläthanolamin/Triäthylendiamin im Gew.-Verhältnis 4:1 | [Teile] | 1,6 | 1,6 | 1,6 | - | - | - | - | - | - |
| Desmorapid PP (der Bayer AG) | [Teile] | - | - | - | 0,6 | 0,6 | 0,6 | 0,6 | 0,6 | 0,6 |
| Zusatzmittel SM | [Teile] | - | - | - | 1,8 | 1,8 | 1,8 | 1,8 | 1,8 | 1,8 |
| Wasser | [Teile] | 1 | 1 | 1 | - | - | - | - | - | - |
| Trichlorfluormethan | [Teile] | 16 | 16 | 16 | 23 | 23 | 23 | 23 | 23 | 23 |
| Roh-MDI | Teile | 67 | 67 | 67 | 62 | 62 | 62 | 55 | 55 | 55 |

Tabelle 5

| Beispiele | | 12 | 13 | 14 | 15 | 16 | 17 | 18 | 19 | 20 |
|---|---|---|---|---|---|---|---|---|---|---|
| Startzeit | (sec) | 10 | 9 | 15 | 10 | 9 | 10 | 14 | 8 | 20 |
| Steigzeit | (sec) | 113 | 110 | 170 | 100 | 90 | 180 | 116 | 97 | 300 |
| Raumgewicht | (g/l) | 30,5 | 25,2 | 26,0 | 34,3 | 25,5 | 21,8 | 34,7 | 26,6 | 20,8 |
| Druckfestigkeit | $(N/mm^2)$ | 0,32 | 0,12 | 0,13 | 0,32 | 0,13 | 0,1 | 0,32 | 0,13 | 0,1 |
| Biegefestigkeit | $(N/mm^2)$ | 0,36 | 0,26 | 0,3 | 0,4 | 0,27 | 0,25 | 0,48 | 0,28 | 0,21 |
| Durchbiegung | (mm) | 14,2 | 14,1 | 17,2 | 13,7 | 13,5 | 16,3 | 13,8 | 12,0 | 17 |

O.Z. 0050/032622

C000468

Patentansprüche

1. Chlor- und estergruppenhaltige Polyole, hergestellt durch Alkoxylierung eines Tetrachlorphthalsäurehalbesters mit einer Viskosisät von 30 bis 20 000 m.Pa.s, gemessen bei 130°C, mit Alkylenoxiden im Äquivalenzverhältnis von Carboxylgruppen zu Alkylenoxid von 1:1 bis 1,5, wobei der Tetrachlorphthalsäurehalbester seinerseits erhalten wird durch Umsetzung von Tetrachlorphthalsäureanhydrid mit

a) einer Mischung aus 2- bis 6-wertigen Alkoholen mit Molekulargewichten von 60 bis 300 oder

b) einer Mischung aus 2- bis 6-funktionellen hydroxylgruppenhaltigen Polyäthern mit einem Molekulargewicht von 100 bis 800 oder

c) einer Mischung aus einem 2- bis 6-wertigen Alkohol mit einem Molekulargewicht von 60 bis 300 und einem 2- bis 6-funktionellen hydroxylgruppenhaltigen Polyäther mit einem Molekulargewicht von 100 bis 800

mit der Maßgabe, daß die Mischung eine Funktionalität von 2,8 bis 4,5 besitzt und das Äquivalenzgewichtsverhältnis von Hydroxyl- zu Anhydridgruppen 1,1 bis 1:1 ist.

2. Chlor- und estergruppenhaltige Polyole gemäß Anspruch 1, dadurch gekennzeichnet, daß man als Alkylenoxide Äthylenoxid, Propylenoxid oder deren Gemische verwendet.

0000468

3. Verfahren zur Herstellung von chlor- und estergruppenhaltigen Polyolen durch Alkoxylierung eines Tetrachlorphthalsäurehalbesters mit Alkylenoxiden, dadurch gekennzeichnet, daß man einen Tetrachlorphthalsäurehalbester mit einer Viskosität von 30 bis 20 000 m.Pa.s, gemessen bei 130°C, erhalten durch Reaktion von Tetrachlorphthalsäureanhydrid

a) einer Mischung aus 2- bis 6-wertigen Alkoholen mit Molekulargewichten von 60 bis 300 oder

b) einer Mischung aus 2- bis 6-funktionellen hydroxylgruppenhaltigen Polyäthern mit einem Molekulargewicht von 100 bis 800 oder

c) einer Mischung aus einem 2- bis 6-wertigen Alkohol mit einem Molekulargewicht von 60 bis 300 und einem 2- bis 6-funktionellen hydroxylgruppenhaltigen Polyäther mit einem Molekulargewicht von 100 bis 800

mit der Maßgabe, daß die Mischung eine Funktionalität von 2,8 bis 4,5 besitzt und das Äquivalenzgewichtsverhältnis von Hydroxyl- zu Anhydridgruppe 1,1 bis 1:1 beträgt mit Alkylenoxiden im Äquivalenzverhältnis von Tetrachlorphthalsäurehalbester zu Alkylenoxid von 1:1,0 bis 1,5 umsetzt.

4. Verfahren gemäß Anspruch 3, dadurch gekennzeichnet, daß man als Alkylenoxide Äthylenoxid, Propylenoxid oder deren Gemische verwendet.

5. Verwendung von chlor- und estergruppenhaltigen Polyolen gemäß Anspruch 1 zur Herstellung vom flammbeständigen Polyurethan- oder Polyisocyanuratschaumstoffen.

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 3 585 185 (W.W.LEVIS)<br>* Spalte 1, Zeilen 12-17; Spalte 2, Zeilen 3-9, 27-43, 49-50, 70-72; Spalte 3, Zeilen 1-21 *<br>----- | 1-5 |

**KLASSIFIKATION DER ANMELDUNG (Int.Cl.²)**

C 07 C 69/80
C 08 K 5/12

**RECHERCHIERTE SACHGEBIETE (Int. Cl.²)**

C 07 C 69/76
C 07 C 69/80
C 07 C 67/26

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 01-09-1978 | KINZINGER |